# EUROPEAN PATENT APPLICATION

(11) **EP 1 099 451 A2**
(43) Date of publication of application: **16.05.2001**
(21) Application number: 00308995.0
(22) Date of filing: 12.10.2000
(51) Int. Cl.: A61M 16/04

(54) **Tracheostomy tube assemblies and obturators**

(30) Priority: 11.11.1999 GB 9926570
(71) Applicant: Smiths Industries Public Limited Company, London, NW11 8DS (GB)
(72) Inventor: Nash, John Edward, Hythe, Kent CT21 6QU (GB); Pagan, Eric, Hythe, Kent CT21 6BD (GB)
(74) Representative: Flint, Jonathan McNeill

(57) **Abstract**

An obturator 2, 2' for a tracheostomy tube 1 has a handle 25, 25' and an enlarged, hollow patient end 21, 21' divided between a tapering forward section 22, 22' and a rear section 23, 23' having a constant, oval cross section. The obturator 2, 2' is inserted in the tracheostomy tube 1 so that the tapering section 22, 22' is located forwardly of the patient end 11 and the oval section 23, 23' extends through the patient end of the tube. The oval shape of the obturator 2, 2' provides a smooth transition between the obturator and the patient end 11 of the tube 1 because the patient end of the tube is distorted to an oval shape when it is straightened by insertion of an obturator.

## Description

This invention relates to tracheostomy tube assemblies of the kind comprising a tracheostomy tube and an obturator, the tracheostomy tube being curved along its length and the obturator being straighter than the tube such that the curve of the tube is reduced when the obturator is inserted and the patient end of the tube is distorted from a circular to a non-circular section.

Tracheostomy tubes are curved tubes used to provide ventilation of a patient via a surgically-made opening through the throat into the trachea. The patient end of the tracheostomy tube is located within the trachea and its machine end protrudes externally from the opening where it may be connected to ventilation apparatus, or left open if the patient is breathing spontaneously. The tracheostomy tube is usually inserted through the opening into the trachea using an obturator. The patient end of the obturator is pointed and projects from the patient end of the tube so as to provide a lead in for the tube, separating the stiff tissues around the opening. The obturator is usually fairly stiff because an appreciable force is often necessary during insertion of the assembly of the tube and obturator; the obturator is also usually curved with a larger radius than the tube so that the assembly is straightened for easier insertion. As a result of this straightening, the tracheostomy tube is deformed at its patient end from a circular section into an oval section, leaving a hollow cusp or cavity between the inside edge of the tracheostomy tube and the obturator. This can lead to tissue damage during insertion.

It is an object of the present invention to provide an alternative tracheostomy assembly and obturator.

According to one aspect of the present invention there is provided a tracheostomy assembly of the above-specified kind, characterised in that the obturator has a patient end with a non-circular section where it extends through the patient end of the tube such as substantially to fill the distorted shape of the patient end of the tube thereby to provide a substantially smooth transition between the patient end of the tube and the obturator around its circumference.

The cross-section of the patient end of the obturator is preferably oval. The patient end of the obturator may comprise a forward section and a rear section, the forward section having a tapered shape located externally of the tracheostomy tube and the rear section extending through the patient end of the tube and providing the non-circular section. The patient end of the obturator is preferably hollow and opens through an aperture at its patient end. The obturator preferably has a section of reduced cross section to the rear of its patient end so as to reduce friction with the inside of the tube. The section of reduced cross section may provide a handle of the obturator. The patient end of the obturator may be collapsible radially during insertion and may be expandable when located in the patient end of the tube. The patient end of the obturator may have lines of weakness extending longitudinally.

According to another aspect of the present invention there is provided an obturator for a tracheostomy tube, the obturator being straighter than the tube in its natural state such that the tube is bent straighter when the obturator is inserted, characterised in that the obturator has a narrow handle forming a major part of its length and an enlarged patient end adapted to make a close sliding fit within the tracheostomy tube, and that the patient end has a section of non-circular section selected to conform substantially to the internal shape of the patient end of the tube when the tube is distorted from a circular shape by the insertion of the obturator such as thereby to provide a substantially smooth transition between the patient end of the tube and the obturator around its circumference.

An assembly of a tracheostomy tube and obturator according to the present invention will now be described, by way of example, with reference to the accompanying drawings, in which:
- Figure 1: is a side elevation view of a tracheostomy tube without an obturator;
- Figure 2: is a side elevation view of the tracheostomy tube assembly with the obturator inserted in the tube;
- Figure 3: is a perspective view of the obturator;
- Figure 4: is an end view of the patient end of the assembly along the arrow IV; and
- Figure 5: is a perspective view of a modified obturator.

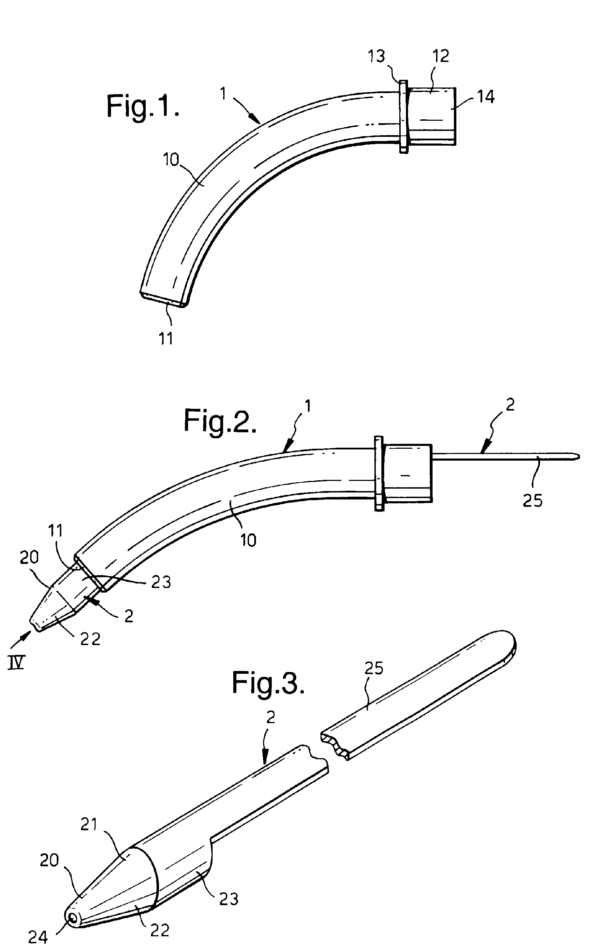

With reference first to Figure 1 there is shown a conventional tracheostomy tube 1 having a hollow shaft 10 of a bendable plastics material, which is curved along its length with a constant radius into a continuous arc. The shaft 10 has a circular cross section along its length, its patient end 11 being tapered externally. At its machine end 12, the tube has a flange 13 and a coupling 14. The tube may have an inflatable cuff towards its patient end, not provided in this embodiment.

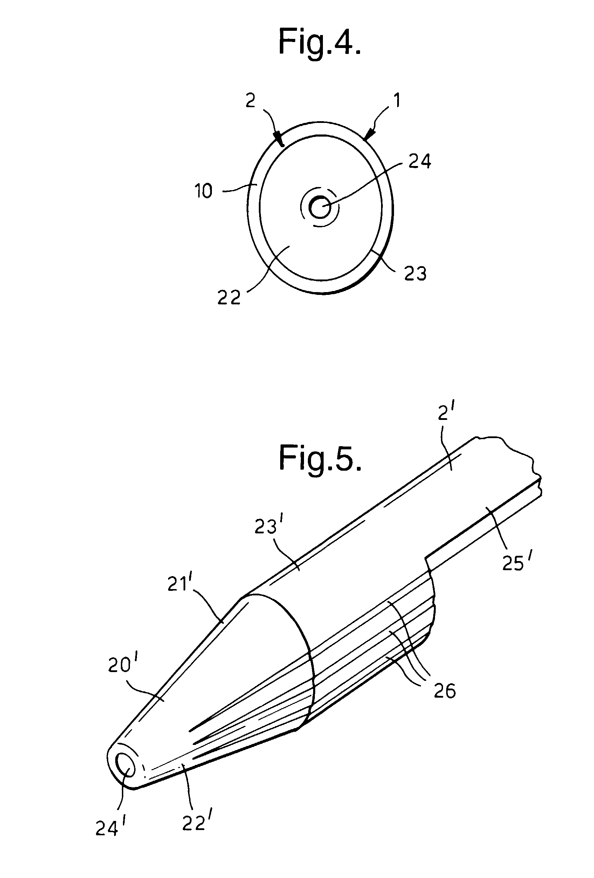

With reference now to Figures 2 to 4, the assembly also includes an obturator 2 moulded from a relatively stiff plastics material. At its patient end 20, the obturator 2 has a hollow portion 21 shaped to fit closely within the patient end 11 of the tube 1. In particular, the patient end portion 21 comprises a forward, tapering section 22 and a rear section 23 of constant cross section along its length. The cross-section of the rear section 23 is shown most clearly in Figure 4 and is non-circular, being substantially oval in shape. The forward section 22 tapers down from this oval shape at its rear end to a small circular aperture 24 at its tip. The aperture 24 opens into the hollow interior of the patient end portion 21 so as to enable the assembly to be threaded on a guide wire (not shown) used for insertion into the trachea. The obturator 2 to the rear of the patient end 20 comprises a solid rod or handle 25 substantially smaller in cross section than the internal section of the shaft 10 of the tube 1 and forming the major part of the length of the obturator. The handle 25 is straight and stiff but is bendable and its length is such that it protrudes from the coupling 14 at the machine end 12 of the tube 1 when inserted such that the patient end 20 of the obturator 2 protrudes from the patient end 11 of the tube.

In use, the obturator 2 is inserted through the machine end 12 of the tube 1 and, as the patient end 20 is pushed along the tube, it will deform the shaft 10 of the tube into an oval shape corresponding to the external shape of the rear section 23 at the patient end of the obturator. The straight shape of the obturator handle 25 straightens the shaft 10 of the tube 1 as the obturator 2 is inserted, although the flexibility of the obturator allows the tube to maintain a curve having a larger radius than that of the tube in its natural state. The straightening of the shaft 10 of the tube I, however, is sufficient to cause its cross section to be distorted from its natural circular shape to an oval shape such that, if the obturator had a circular cross section, there would be a hollow cusp or cavity between the tube and obturator. The external shape of the rear section 23 of the patient end of the obturator 2 is chosen to fill this shape, so that there is a smooth transition between the tapered end 11 of the tube 1 and the external surface of the obturator. The obturator 2 is inserted until the patient end 11 of the tube 1 is to the rear of the tapering section 22 and about midway along the rear section 23 so that the tapering section is located external of the tube. The non-circular section 23 of the patient end of the obturator 2 produces a relatively high friction between the obturator and inside of the shaft 10 of the tube 1 but the overall effect of this does not cause difficulty in inserting the obturator because of the short length of the patient end portion.

With reference to Figure 5, the patient end portion of an obturator 2' could be arranged to collapse radially to a circular shape when being inserted along a circular portion of the tube, but to expand to its natural oval shape when located in the deformed patient end of the tube. The patient end portion 20' of the obturator 2' could be made collapsible in this way by means of lines of weakness 26 extending longitudinally of the patient end portion, the lines of weakness being, for example, lines of reduced thickness.

The arrangement of the present invention provides a tracheostomy tube assembly that can be inserted with a reduced risk of tissue damage.

## Claims

1. A tracheostomy assembly comprising a tracheostomy tube (1) and an obturator (2, 2'), the tracheostomy tube being curved along its length and the obturator being straighter than the tube such that the curve of the tube is reduced when the obturator is inserted and the patient end (11) of the tube is distorted from a circular to a non-circular section, characterised in that the obturator (2, 2') has a patient end (21, 21') with a non-circular section (23, 23') where it extends through the patient end (11) of the tube (1) such as substantially to fill the distorted shape of the patient end of the tube thereby to provide a substantially smooth transition between the patient end (11) of the tube and the obturator (2, 2') around its circumference.

2. An assembly according to Claim 1, characterised in that the cross-section of the patient end (21, 21') of the obturator (2, 2') is oval.

3. An assembly according to Claim 1 or 2, characterised in that the patient end (21, 21') of the obturator (7, 2') comprises a forward section (22, 22') and a rear section (23, 23'), that the forward section (22, 22') has a tapered shape located externally of the tracheostomy tube (1), and that the rear section (23, 23') extends through the patient end (11) of the tube (1) and provides the non-circular section.

4. An assembly according to any one of the preceding claims, characterised in that the patient end (21, 21') of the obturator (2, 2') is hollow and opens through an aperture (24, 24') at its patient end.

5. An assembly according to any one of the preceding claims, characterised in that the obturator (2, 2') has a section (25, 25') of reduced cross section to the rear of its patient end (71, 71') so as to reduce friction with the inside of the tube (1).

6. An assembly according to Claim 5, characterised in that the section (25, 25') of reduced cross section provides a handle of the obturator (2, 2').

7. An assembly according to any one of the preceding claims, characterised in that the patient end (21') of the obturator (2') is collapsible radially during insertion and is expandable when located in the patient end of the tube (1).

8. An assembly according to Claim 7, characterised in that the patient end (21') of the obturator (2') has lines weakness (26) extending longitudinally.

9. An obturator (2, 2') for a tracheostomy tube (1), the obturator being straighter than the tube in its natural state such that the tube is bent straighter when the obturator is inserted, characterised in that the obturator (2, 2') has a narrow handle (25, 25') forming a major part of its length and an enlarged patient end (21, 21') adapted to make a close sliding fit within the tracheostomy tube (1), and that the patient end (21, 21') has a section (23, 23') of non-circular section selected to conform substantially to the internal shape of the patient end (11) of the tube (1) when the tube is distorted from a circular shape by the insertion of the obturator such as thereby to provide a substantially smooth transition between the patient end (11) of the tube (1) and the obturator (2, 2') around its circumference.
